# EUROPEAN PATENT APPLICATION

(11) **EP 1 832 228 A1**
(43) Date of publication of application: **12.09.2007**
(21) Application number: 05824393.2
(22) Date of filing: 01.12.2005
(51) Int. Cl.: A61B 5/053, A61B 5/103

(54) **METHOD OF EVALUATING AND CONTROLLING THE DEGREE OF VASCULARISATION IN PARTS OF THE HUMAN BODY AND DEVICE FOR IMPLEMENTING SAME**

(30) Priority: 17.12.2004 ES 200403001
(71) Applicant: INDIBA, S.A., E-08013 Barcelona (ES)
(72) Inventor: CALBET BENACH, José, c/o Indiba, S.A., E-08009 Barcelona (ES)
(74) Representative: Pastells Teixido, Manuel
(86) International application number: PCT/ES2005/000657
(87) International publication number: WO 2006/067246

(57) **Abstract**

The invention relates to a method of evaluating and controlling the degree of vascularization in parts of the human body and to a device for implementing same. The inventive method comprises the following steps consisting in: installing conductive electrodes in the part of the human body comprising the zone for which vascularization is to be evaluated; passing a high-frequency current between the electrodes; measuring and recording the values of the circulating electric intensity; and comparing the recorded through-current values with constant values in order to evaluate the vascularization. The inventive device comprises two electrodes which are connected to high-frequency generator and which are connected to a high--frequency generator and which are applied to a measuring apparatus which displays the degree of vascularization and which is provide with a milliammeter for measuring the through-current.

## Description

### OBJECT OF THE INVENTION

A process for assessing and controlling the vascularization degree in portions of the human body, and a device for carrying out this process.

### BACKGROUND OF THE INVENTION

There exists nowadays a problem at a worldwide level when in a hospital or clinic it becomes necessary to measure the blood circulation or vascularization level in a patient in order to ascertain his or her condition, so as to thus be in a position to determine if he or she has to be put under treatment in order to improve the vascularization.

The methods being so far known for assessing the vascularization are:
- Angiography, which is an invasive method.
- Plethysmography or blood flow detection at the fingers; and
- Capillarioscopy or capillary count at the fingers. These two last methods do not provide a very accurate assessment of the vascularization of the limb.
- Echo-Doppler, which is a method that provides an orientative but nonquantitative information about the vascularization of the limb.
- Laser-Doppler, which is the method that provides the most accurate assessment even if only at a surface level.
- Thermography (infrared), which is only orientative.
   The invasive method has an evidently limited massive application.
   The aforementioned noninvasive methods do not allow an assessment of the vascularization in volumes of tissue since they do not go deeply into the tissue.
   The knowledge of the degree of vascularization is important both for the diagnosis and for the followup of the evolution of the treatments of many infirmities, and also for ascertaining the symmetry ratio between the two sides of the body. There is hence a problem of lack of adequate means allowing to easily assess the degree of vascularization.

### CHARACTERIZING FEATURES OF THE INVENTION

It is the object of this invention to solve this problem, and for such a purpose multiple tests and experiments have been carried out by a panel of vascularization experts and have finally resulted in the process being the object of the present invention and in the device for carrying it out.

The new process being based on the knowledge that the conductivity of human tissues increases as a function of their hematic content, and hence with the increase of the vascularization, comprises the following successive stages:
a) Installing electroconductive electrodes in portions of the human body comprising the region whose vascularization is to be assessed.
   - The location of the electrodes must be exactly the same in the different tests.
b) Passing a high-frequency current between the two electrodes or electrode groups without reaching the point of thereby causing a temperature increase in the human tissue.
c) Measuring and recording the flowing current values for an applied tension value or for different applied tension values.
   - The impedance or the admittance can be computed if necessary.
d) Comparing the recorded values with previously obtained standard values so as to be in a position to assess the vascularization of the limb or body region being examined.
   - The assessment of the obtained data will be always carried out by comparing them with previous readings having been obtained with the same arrangement of the electrodes and the same applied tension and frequency values, or by comparing them with those of symmetric regions of the same patient while equally respecting the symmetry in the arrangement of the applied electrodes and the applied tension and frequency values.
   - A table of values can be obtained with the results, these latter being also plotted in graph form.

This process allows to very accurately detect the improvement ratio between the first treatment and the following ones both and irrespective of if the treatments are carried out with apparatuses such as hyperthermia units and if they are carried out with drugs.

The device for carrying out the aforementioned process comprises two electrodes that while being connected to a high-frequency generator are applied to a measuring unit being fit to display the vascularization degree readout, said unit being also provided with a milliammeter that will measure the electric current flowing through.

This device has been shielded so as to prevent the flowing high-frequency current from affecting the very measuring unit.

The applied electrodes can have any geometry, and their surface contacting the skin is an electroconductive surface.

Said electrodes can be manually applicable, adhesive and separate, or else they can be mechanically connected with each other and thus form an assembly in order to thus enhance the repeatability of the tests.

The generator must produce an alternating current having a frequency of between 0.150 and 10 MHz at a controllable tension ranging from 0,1 to 50 volts. Said high-frequency generator can be incorporated into the measuring unit, or else it can be external to this latter.

The milliammeter has a scale bottom value of between 10 µA and 1,000 mA with a resolution ranging between 0. 1 µA and 1 mA.

Alternatively the same unit or independently another instrument will measure the applied tension.

A computer can be optionally incorporated into the device in order to compute the data having been obtained with this latter and other data of the patient, such as his or her historical record, the type of infirmity, the evolution and other features.

This device for assessing the vascularization changes can be included in any newly designed or already existing therapeutic unit affecting said vascularization, such as for example microwave, short-wave, electrical stimulation, ultrasound, hyperthermia and other units.

These and other features will be best made apparent by the following detailed description whose understanding will be made easier by the accompanying three sheets of drawings showing a practical embodiment being cited only by way of example not limiting the scope of the present invention.

### DESCRIPTION OF THE DRAWINGS

In the drawings:
Figs. 1 through 5 diagrammatically represent respective variations of the process entailing the application of the assessing device to different regions of the patient's body;
Fig. 6 diagrammatically illustrates a variation of the device wherein the contact electrodes are mechanically connected with each other;
Fig. 7 diagrammatically shows the device having been incorporated into a therapeutic, treating unit; and
Figs. 8, 9 and 10 respectively represent values having been measured on a leg and an arm, as well as measurements having been carried out on a patient suffering from low back pain in the left side.

### DETAILED DESCRIPTION

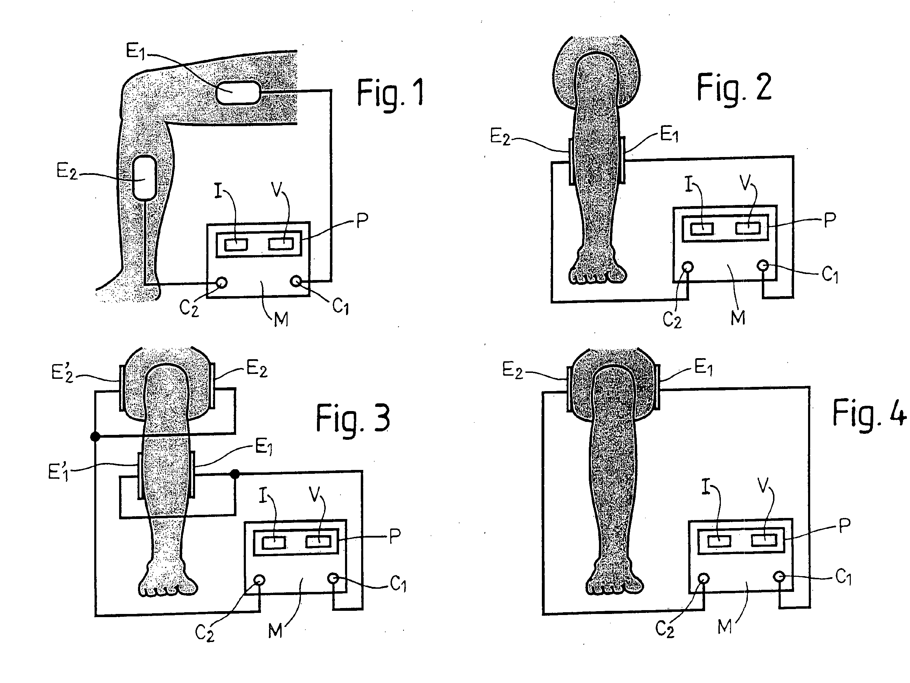

In Fig. 1 the measuring device (M) comprises a display (P) being provided for displaying the result of the measurement and thereby showing the vascularization current (I) and the applied tension as voltage (V), said measuring device also having two connections namely being the one being shown at (C1) for an electrode (E1) being applied to the patient's thigh, and the one being shown at (C2) for an electrode (E2) being applied to said patient's leg.

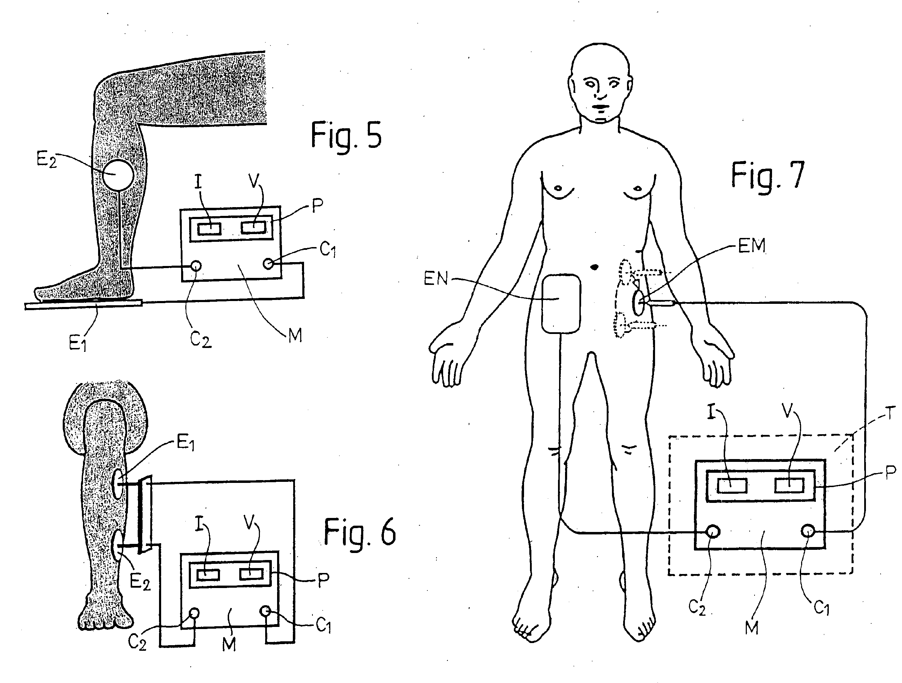

In Fig. 2 the electrodes (E1) and (E2) are both applied to the leg on opposite sides of this latter. In Fig. 3 the connection (C1) serves two electrodes (E1) and (E'1) being each applied to one of the opposite sides of the leg, and the connection (C2) serves two electrodes (E2) and (E'2) being each applied to one of the opposite sides of the thigh. In Fig. 4 the electrodes (E1) and (E2) are both applied to the thigh, each of them being namely applied to one of the opposite sides of said thigh. In Fig. 5 the electrode (E1) is applied to the foot sole, and the electrode (E2) is applied to one side of the leg. In these cases the electrodes will be shaped as befits the anatomy of the region to be analyzed in the patient. The electrodes must be asymmetric when carrying out a therapeutic treatment on top of controlling the degree of vascularization.

In Fig. 7 the measuring device (M) is incorporated into a therapeutic unit (T) for an hyperthermia treatment, an active, metallic electrode (EM) being connected to connection (C1) and shiftable on the patient's region to be treated, and a stationary, plate-shaped, neutral or return electrode (EN) is connected to the connection (C2), the patient's body in such an arrangement acting as a resistive element.

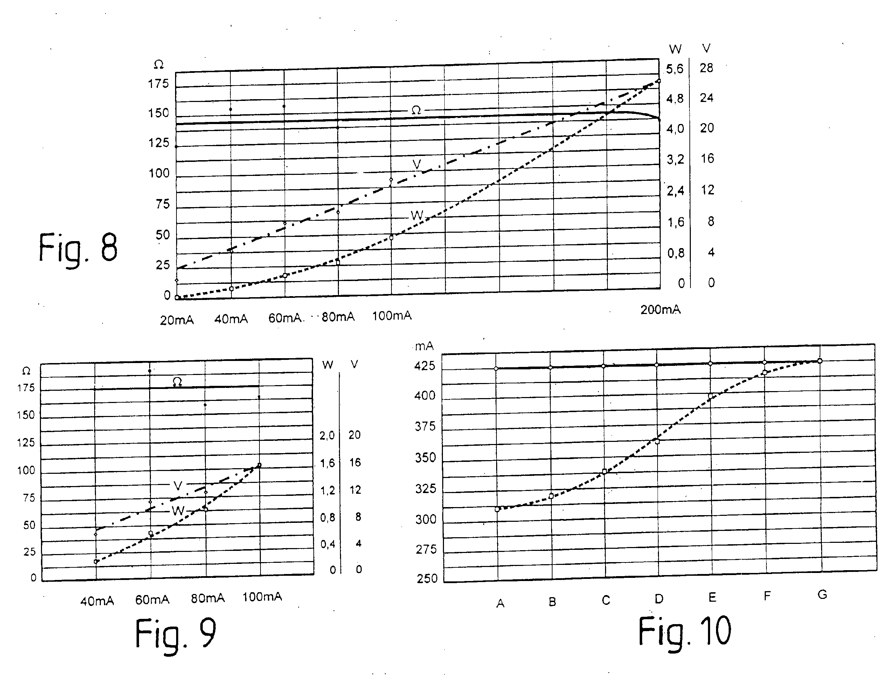

The plot of Fig. 8 shows some possible limits for the advisedly lowest and highest current levels that can be used for the athermal measurements of the vascularization level, said levels being selected by choosing the adequate tension of the generator. Said plot allows to appreciate the voltage and current values, the watts being dissipated at each point and the corresponding resistance or impedance as observed on a healthy leg and based on the contact electrodes having remained in the same physical location during all of the measurements. Under the same conditions Fig. 9 shows the values having been obtained on an arm.

The plot of Fig. 10 allows to observe the relationship existing between the current difference or increase after each treatment in a problem of acute low back pain being treated with hyperthermia, up to the total asymptomatology after the 6^{th} treatment. The solid line shows the data from the readings having been obtained in the healthy right hand side region. The dash line shows the data from the readings having been obtained in the affected left hand side region and evidencing the process. On the abscissae axis the points A, B, C, D, E, F and G have been represented and respectively correspond to the situation before and after the 1^{st} to 6^{th} treatments.

The following Tables I, II and III respectively correspond to the values of Figs. 8, 9 and 10.

**TABLE I**

| Applied current mA | Measured tension V | Power output W | Resulting impedance Ω |
|---|---|---|---|
| 20 | 2.5 | 0.050 | 125 |
| 40 | 6.2 | 0.248 | 155 |
| 60 | 9.4 | 0.564 | 156.5 |
| 80 | 11 | 0.880 | 137.5 |
| 100 | 15 | 1.500 | 150 |
| 200 | 27 | 5.4 | 135 |

**TABLE II**

| Applied current mA | Measured tension V | Power output W | Resulting impedance Ω |
|---|---|---|---|
| 40 | 7 | 0.280 | 175 |
| 60 | 11.5 | 0.690 | 191.5 |
| 80 | 12.7 | 1.016 | 158.5 |
| 100 | 16.5 | 1.650 | 165 |

**TABLA III**

| | Applied voltage V | Affected low back region | Healthy low back region |
|---|---|---|---|
| | | Measured current in mA | Measured current in mA |
| Before starting the treatment | 22 | 310 | 425 |
| After the 1^{st} treatment | 22 | 318 | 425 |
| After the 2^{nd} treatment | 22 | 338 | 425 |
| After the 3^{d} treatment | 22 | 367 | 425 |
| After the 4^{th} treatment | 22 | 396 | 425 |
| After the 5^{th} treatment | 22 | 417 | 425 |
| After the 6^{th} treatment | 22 | 425 | 425 |

It is important to point out that although a number of electrical measurements have already been carried out at present on the body there is no known precedent of flowing current measurements from a high frequency ranging between 0.1 and 10 MHz having ever been associated with a given level of vascularization.

This new process for assessing and controlling the degree of vascularization in a patient allows to accurately diagnose in advance, i.e. before the patient starts showing the first physical symptoms, where the vascularization problem actually exists, and in case of this latter having already manifested itself the degree of seriousness will be ascertained in each case so as to thus be in a position to later on treat the problem in an adequate manner.

## Claims

1. A process for assessing and controlling the vascularization degree in portions of the human body, **characterized in that** it comprises the following operational stages to be carried out successively:
a) Installing electroconductive electrodes in portions of the human body comprising the region whose vascularization is to be assessed.
b) Passing a high-frequency current between the electrodes without causing a temperature increase in the human tissue.
c) Measuring and recording the flowing current values for an applied tension value or for different applied tension values; and
d) Comparing the recorded through-flowing current values with standard values so as to be in a position to assess the vascularization of the limb or body region being examined.

2. A process as per claim 1, **characterized in that** the recorded current values are compared with previous readings having been obtained with the same arrangement of the electrodes and the same applied tension and frequency values.

3. A process as per claim 1, **characterized in that** the current values are compared with those of symmetric regions of the body of the same patient.

4. A device for carrying out the process as per claim 1, **characterized in that** it comprises two electrodes that while being connected to a high-frequency generator are applied to a measuring unit being fit to display the vascularization degree readout, said unit being also provided with a milliammeter that will measure the electric current flowing through.

5. A device as per claim 4, **characterized in that** it comprises means for setting the voltage in accordance with the body portion whose vascularization is to be assessed.

6. A device as per claim 4, **characterized in that** the electric current being applied between the electrodes is an alternating current having a frequency ranging between 0.150 and 10 MHz at a controllable tension ranging from 0,1 and 50 volts.

7. A device as per claim 4, **characterized in that** the milliammeter has a scale bottom value of between 10 µA and 1,000 mA with a resolution ranging between 0. 1 µA and 1 mA.

8. A device as per claim 4, **characterized in that** it comprises a computer into which all of the patient's data will be entered together with the control of his or her evolution.
